# EUROPEAN PATENT APPLICATION

(11) **EP 0 838 199 A1**
(43) Date of publication of application: **29.04.1998**
(21) Application number: 97117950.2
(22) Date of filing: 16.10.1997
(51) Int. Cl.: A61B 17/74, A61B 17/72

(54) **Nail for reducing fractures of the trochanter and of the neck of the femur**

(30) Priority: 24.10.1996 IT BO960146 U
(71) Applicant: Hit Medica S.r.l., 47037 Rimini (IT)
(72) Inventor: Iaia, Ciro Rosario, 47037 Rimini (IT)
(74) Representative: Modiano, Guido, Dr.-Ing.

(57) **Abstract**

A nail for reducing fractures of the trochanter and of the neck of the femur, comprising an elongated and angled shank (2) in which, proximate to the upper end, a head screw (5) can be inserted downward from above and can be locked to the nail by a screw (8) which is screwed into a threaded axial hole (9) of the top of the shank (2); the shank has, in order to facilitate insertion in the medullary canal and reduce and better distribute the mechanical stresses affecting the bone, a lower end (3) which is cut along an inclined plane like a flute mouthpiece and has, proximate to a hole (4) for the insertion of the head screw (5), a through hole (11) which has a reduced diameter and is substantially parallel to the hole of the head screw (5), for the passage of a wire (12) which is threaded at its top end (13) for temporary screwing in the head of the femur (F) and is adapted to avoid rotations of the head of the femur during the screwing of the head screw (5).

## Description

The present invention relates to a nail for reducing fractures of the trochanter and of the neck of the femur, particularly pertrochanteric, subtrochanteric, intertrochanteric fractures and fractures of the neck of the femur.

Fractures of the trochanter and of the neck of the femur are reduced by using steel nails constituted by an elongated and angled shank which has, proximate to its upper end, a slanted inclined through hole in which a so-called head screw can be inserted downward from above; the top of the head screw is meant to be screwed into the head of the femur, and the head screw can be locked onto the nail by a screw which is screwed into a threaded axial hole of the top of the shank; in the lower region of the shank there are provided two diametrical holes wherein respective so-called distal screws can be screwed in order to fix the base of the shank to the base of the femur.

Those nails entail some drawbacks in relation to the angled arrangement of the shank, which forces to drill the femur with bores which have an excessive diameter with respect to the diameter of the shank, and to the stubby shape of the lower end of the shank, which forms a rather sudden truncation which can produce a preferential bone breakage region; moreover, torsional stresses often affect the head of the femur during the tightening of the head screw and there are problems in relation to the material that is normally used, usually highly rigid stainless steel, which generates sometimes traumatic stresses in the bone of the femur.

The aim of the present invention is to obviate the cited drawbacks of conventional devices, i.e., to provide a nail for reducing fractures of the trochanter and of the neck of the femur the installation whereof entails drilling holes whose diameter is only slightly greater than the diameter of the shank, does not entail a preferential bone breakage region, does not generate torsional stresses in the femur during the tightening of the head screw and is made of a material whose elasticity is comparable to that of a bone.

Within the scope of this aim, another object of the present invention is to provide a structure which is simple, relatively easy to provide in practice, safe in use, effective in operation and relatively modest in cost.

This aim, these objects and others which will become apparent hereinafter are achieved by a nail for reducing fractures of the trochanter and of the neck of the femur, of the kind constituted by an elongated and angled shank in which, proximate to the upper end, a head screw can be inserted downward from above and can be locked to the nail by a screw which is screwed into a threaded axial hole of the top of the shank, characterized in that the shank has, in order to facilitate insertion in the medullary canal and reduce and better distribute the mechanical stresses affecting the bone, a lower end which is cut along an inclined plane like a flute mouthpiece and has, proximate to a hole for the insertion of said head screw, a through hole which has a reduced diameter and is substantially parallel to said hole of the head screw, for the passage of a wire which is threaded at its top end for temporary screwing in the head of the femur and is adapted to avoid rotations of the head of the femur during the screwing of the head screw.

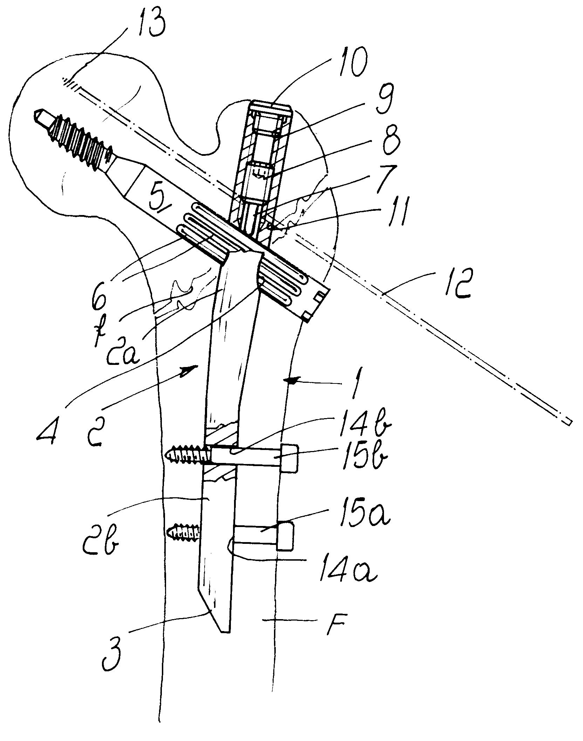

Further characteristics and advantages of the present invention will become apparent from the following detailed description of a preferred but not exclusive embodiment of a nail for reducing fractures of the trochanter and of the neck of the femur according to the present invention, illustrated only by way of non-limitative example in the accompanying drawings, wherein:
the only figure is a partially cutout side view of a nail for reducing fractures of the trochanter and of the neck of the femur according to the present invention.

With particular reference to the above figure, the reference numeral 1 generally designates a nail for reducing fractures f of the trochanter and of the neck of the femur F according to the present invention.

The nail 1 is of the kind constituted by an elongated shank 2 which has a cylindrical cross-section and is constituted by an upper part 2a and by a lower part 2b which has a slightly reduced diameter; these parts form, with respect to each other, an angle of six degrees so as to reduce the bone drilling diameter for its insertion.

The lower end 3 of the shank is cut along an inclined plane, i.e., like a flute mouthpiece, in order to facilitate insertion in the medullary canal and better distribute the mechanical stresses affecting the bone.

Proximate to its upper end, the shank 2a is crossed by an oblique hole 4 in which it is possible to insert upward, from below, a head screw 5 which has, in its lower region, eight rotation-preventing longitudinal grooves 6 for the selective insertion of the tip 7 of a locking screw 8 which is screwed into a threaded axial hole 9 of the top of the shank.

A closure plug 10 is screwed into the upper end of the axial hole 9 of the shank and is used to prevent, during the healing process, any accumulation of blood and/or bone calcification, which should be removed for shank extraction.

Proximate to the hole 4 for the insertion of the head screw 5, the shank 2 is crossed by a small through hole 11 which has a reduced diameter and is substantially parallel to the hole 4: a steel wire 12, threaded at a top end 13 (known in the field as a Kirschner's wire), can be inserted in the hole 11 for temporary screwing in the head of the femur F during the screwing of the head screw 5; this wire 12 is adapted to prevent rotations of the head of the femur during the screwing of the head screw 5 and is removed once fixing has occurred.

Two diametrical holes 14a, 14b are provided in the lower region 2b of the shank 2, and two conventional screws 15a, 15b can be inserted in said holes and allow to fix the lower part 2b of the shank 2 to the bone of the femur F.

Advantageously, all the components of the nail 1 are made of titanium alloy, which has never been used for the specific kind of nail, is biocompatible, allows to perform magnetic resonance and has an elasticity modulus which is close to that of a bone, so as to make the implant more elastic than steel in order to reduce the possibility of bone damage.

It is thus evident that the present invention achieves the intended aim and object.

The invention thus conceived is susceptible of numerous modifications and variations, all of which are within the scope of the inventive concept.

All the details may also be replaced with other technically equivalent ones.

In practice, the materials employed, as well as the shapes and the dimensions, may be any according to the requirements without thereby abandoning the scope of the protection of the appended claims.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A nail for reducing fractures (f) of the trochanter and of the neck of the femur (F), comprising an elongated and angled shank (2) in which, proximate to the upper end, a head screw (5) can be inserted downward from above and can be locked to the nail by means of a screw (8) which is screwed into a threaded axial hole (9) of the top of the shank, characterized in that the shank (2) has, in order to facilitate insertion in the medullary canal and reduce and better distribute the mechanical stresses affecting the bone, a lower end (3) which is cut along an inclined plane like a flute mouthpiece and has, proximate to a hole (4) for the insertion of said head screw, a through hole (11) which has a reduced diameter and is substantially parallel to said hole of the head screw (5), for the passage of a wire (12) which is threaded at its top end (13) for temporary screwing in the head of the femur (F) and is adapted to avoid rotations of the head of the femur during the screwing of the head screw (5).

2. A nail according to claim 1, characterized in that said shank (2) is constituted by an upper part (2a) and by a lower part (2b) which form, between them, an angle of six degrees which is adapted to reduce the bone drilling diameter.

3. A nail according to claim 1, characterized in that the components are made of titanium alloy, which is biocompatible, allows to perform magnetic resonance and is adapted to make the implant more elastic than steel in order to reduce the possibility of bone damage.

4. A nail according to claim 2, characterized in that said head screw (5) has, in the lower region, eight longitudinal rotation-preventing grooves (6) for the selective insertion of a tip (7) of said screw (8) screwed into the axial hole (9) of said shank (2).
